(19) Europäisches Patentamt
European Patent Office
Office européen des brevets

(11) EP 0 586 344 B1

(12) EUROPÄISCHE PATENTSCHRIFT

(45) Veröffentlichungstag und Bekanntmachung des Hinweises auf die Patenterteilung:
**09.09.1998 Patentblatt 1998/37**

(51) Int. Cl.⁶: **C07D 263/06**, C07D 263/52, A61K 31/42

(21) Anmeldenummer: **93810598.8**

(22) Anmeldetag: **24.08.1993**

# (54) Oxazolidin Verbindungen und ihre Verwendung als Arzneimittel

Oxazolidene derivatives and their use as pharmaceuticals

Dérivés d'oxazolidins et leur utilisation comme médicaments

(84) Benannte Vertragsstaaten:
**AT BE CH DE DK ES FR GB GR IE IT LI LU NL PT SE**

(30) Priorität: **02.09.1992 CH 2742/92**

(43) Veröffentlichungstag der Anmeldung:
**09.03.1994 Patentblatt 1994/10**

(73) Patentinhaber: **Novartis AG**
**4058 Basel (CH)**

(72) Erfinder:
- **Sallmann, Alfred, Dr.**
  **CH-4103 Bottmingen (CH)**
- **Gschwind, Hans-Peter, Dr.**
  **CH-4056 Basel (CH)**
- **Francotte, Eric, Dr.**
  **CH-4412 Nuglar (CH)**

(56) Entgegenhaltungen:
**DE-A- 2 305 092**

- **JOURNAL OF PHARMACEUTICAL SCIENCES Bd. 72, Nr. 11 , November 1983 , WASHINGTON US Seiten 1294 - 1298 MARIANNE JOHANSEN ET AL 'Prodrugs as drug delivery systems XXV:hydrolysis of oxazolidines.A potential new prodrug type'**
- **CHEMICAL AND PHARMACEUTICAL BULLETIN. Bd. 25, Nr. 6 , Juni 1977 , TOKYO JP Seiten 1368 - 1377 KIYOSHI MURASE ET AL 'New beta-adrenoreceptor stimulants.Studies on 3-acylamino-4-hydroxy-alpha-(N-substituted aminomethyl)benzyl alcohols'**

Bemerkungen:
Die Akte enthält technische Angaben, die nach dem Eingang der Anmeldung eingereicht wurden und die nicht in dieser Patentschrift enthalten sind.

EP 0 586 344 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

## Beschreibung

Die Erfindung betrifft neue Oxazolidinverbindungen der Formel(I)

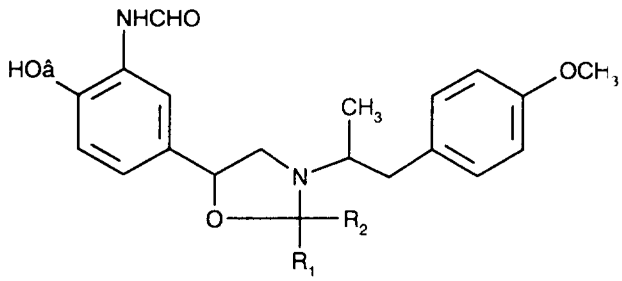

(I)

in welcher $R_1$ und $R_2$ gleichzeitig Wasserstoff, oder beide den gleichen Niederalkylrest mit 1-4 C-Atomen bedeuten oder gemeinsam für 4- bis 7-gliedriges Niederalkylen stehen, und ihre Salze in razemischer und chiraler Form, Verfahren zur Herstellung der genannten Verbindungen, diese enthaltende pharmazeutischen Präparate und ihre Verwendung als Arzneimittelwirkstoffe.

Aus der U.S.-Patentschrift Nr.4,407,819 (American Cyanamid Co.) sind Oxazolidine bekannt, die sich durch die Anordnung der Substituenten am Phenylrest und durch den Substituenten am Stickstoffatom im Oxazolidinring unterscheiden und als Futtermittelzusätze verwendet werden. Aus der DE-A-2305092 sind $\alpha$-Aminomethylbenzylalkoholderivate und aus Chem. Pharm. Bull. 25(6), S.1368-1377 sind $\alpha$-Acylaminobenzylalkoholderivate bekannt, die sich von den vorliegend beanspruchten Verbindungen dadurch unterscheiden, dass sie keine Oxazolidinstruktur als Merkmal enthalten. Beide Verbindungstypen sind als $\beta$-adrenergische Stimulantien verwendbar und damit als bronchodilatorische Mittel geignet. In Journal of Pharmaceutical Science,72, 1983, p.1294 -1298 wird beschrieben, dass Oxazolidinderivate als potentielle Prodrugderivate für entsprechende $\beta$-Aminoalkohole bezeichnet werden können, da diese sich durch quantitative Hydrolyse umgehend umwandeln lassen.

Vor- und nachstehend sind unter niederen Resten und Verbindungen beispielsweise solche zu verstehen, die bis und mit 7, vorzugsweise bis und mit 4 Kohlenstoffatome (C-Atome) aufweisen.
Niederalkyl ist $C_1$-$C_4$-Alkyl, wie Methyl, Ethyl, Propyl oder Butyl. 4- bis 7-gliedriges Niederalkylen ist in erster Linie 1,4-Butylen, 1,5-Pentylen, 1,6-Hexylen,ferner 1,7-Heptylen.
Die erfindungsgemässen Verbindungen umfassen somit alle Enantiomeren, Diastereomeren und ihre Gemische einschliesslich der Razemate. Verbindungen der Formel I im Rahmen der Erfindung liegen bevorzugt als ein Razemat von möglichen Stereoisomeren (R,R und S,S) vor, besonders bevorzugt jedoch in chiralen Formen R,R und S,S.
Salze von Verbindungen sind insbesondere pharmazeutisch verwendbare Salze, z.B. Säureadditionssalze, die beispielsweise mit starken anorganischen Säuren, wie Mineralsäuren, z.B. Schwefelsäure, einer Phosphorsäure oder einer Halogenwasserstoffsäure, mit starken organischen Carbonsäuren, wie Niederalkancarbonsäuren, z.B. Essigsäure, wie gegebenenfalls ungesättigten Dicarbonsäuren, z.B. Malon-, Malein- oder Fumarsäure, oder wie Hydroxycarbonsäuren, z.B. Wein- oder Zitronensäure, oder mit Sulfonsäuren, wie Niederalkan- oder gegebenenfalls substituierten Benzolsulfonsäuren, z.B. Methan- oder p-Toluolsulfonsäure, gebildet werden, oder Salze mit Basen, wie entsprechende Alkalimetall- oder Erdalkalimetallsalze, z.B. Natrium-, Kalium- oder Magnesiumsalze, pharmazeutisch verwendbare Uebergangsmetallsalze, wie Zink- oder Kupfersalze, oder Salze mit Ammoniak oder organischen Aminen, wie cyclischen Aminen, wie Mono-, Di- oder Triniederalkylaminen, wie Hydroxyniederalkylaminen, z.B. Mono-, Di- oder Trihydroxyniederalkylaminen, Hydroxyniederalkylniederalkylaminen oder Polyhydroxyniederalkylaminen. Cyclische Amine sind z.B. Morpholin, Thiomorpholin, Piperidin oder Pyrrolidin. Als Mononiederalkylamine kommen beispielswese Ethyl- und t-Butylamin, als Diniederalkylamine beispielsweise Diethyl- und Diisopropylamin und als Triniederalkylamine beispielsweise Trimethyl- und Triethylamin in Betracht. Entsprechende Hydroxyniederalkylamine sind z.B. Mono-, Di- und Triethanolamin; Hydroxyniederalkyl-niederalkyl-amine sind z.B. N,N-Dimethylamino- und N,N-Diethylaminethanol; als Polyhydroxyniedalkyamin kommt z.B. Glucosamin in Frage. Umfasst sind ferner für pharmazeutische Verwendungen ungeeignete Salze, da diese beispielsweise für die Isolierung bzw. Reinigung freier Verbindungen I sowie deren pharmazeutisch verwendbarer Salze verwendet werden können.

Die Verbindungen I und ihre pharmazeutisch verwendbaren Salze weisen beispielsweise wertvolle pharmakologische Eigenschaften auf.
Die erfindungsgemässen Verbindungen der Formel I besitzen eine langanhaltende stimulierende Wirkung bei Beta-Adrenorezeptoren oder sie bewirken eine Relaxation an empfindlicher glatter Muskulatur.
Aufgrund dieser wirksamen Relaxation an empfindlicher glatter Muskulatur können die Verbindungen der Formel I zur

Verhinderung oder zur Behandlung von Bronchospasmus und Dispnoe bei Erkrankungen wie Bronchialasthma, chronischer Bronchitis und chronischen obstruktiven Lungenerkrankungen, anaphylaktischem Bronchospasmus und cystischer Fibrose sowie zur Verhinderung oder Begrenzung frühzeitiger Wehen im Stadium einer Schwangerschaft verwendet werden.
Die Verbindungen der Formel I sind auch nützlich zur Verhinderung oder Behandlung von inflammatorischen Erscheinungen in einer Vielzahl von Krankheiten, insbesondere, wo die Aktivierung von Beta-Adrenorezeptoren das Erscheinungsbild der Krankheit beeinflussen.
Insbesondere sind die Verbindungen der Formel I geeignet zur Verhinderung oder zur Begrenzung der Freisetzung von vorgeformten oder neu synthetisierten Entzündungsvermittlern, von zellulären Degranulationsprodukten und reaktiven Sauerstoffverbindungen von Zellen wie Mastzellen, Makrophagen, basophilen Zellen, eosinophilen Zellen und Lymphozyten.
Die Verbindungen der Formel I erzeugen eine antiinflammatorische Wirkung durch Verhinderung oder Begrenzung der Freisetzung von Entzündungsvermittlern, wie z.B. Histamin, Leukotrienen, basische und kationische Proteine, Tryptane und Chymase, Zytokine und dergleichen und sind zur Behandlung chronischer und akuter Urticaria, Psoriasis, allergischer Konjunktivitis, Akinitis, Heuschnupfen, Mastocytosis und dergleichen geeignet.

Durch Aktivierung von Endothelial-Beta-adrenorezeptoren sind die erfindungsgemässen Verbindungen auch geeignet, die Folgen und Schäden durch eine erhöhte microvaskuläre Permeabilität zu verhindern oder zu mindern und sind z.B. für Entzündungen, verursacht durch Entzündungsvermittler, chirurgische Eingriffe, Verletzungen, Verbrennungen und Strahlenschäden verwendbar. Die Verbindungen sind daher geeignet bei der Behandlung von Krankheiten verwendet zu werden, welche mit einer Atemwegs-Obstruktion assoziiert sind, wie z.B. Asthma, chronischer Bronchitis und andere Lungenkrankheiten, Schwellungen, Extravasationen durch chirurgische Eingriffe, chemische Verletzungen, Verbrennungen und auch Strahlenschäden, wie z.B. Gehirnödeme und andere Schäden die durch Strahlenbehandlung hervorgerufen werden. Durch Aktivierung des SignaltransduktionMechanismus, der an Beta-Adrenorezeptoren gekoppelt ist, wie z.B. Adenylyl Cyclase (jedoch nicht darauf begrenzt), verhindern die erfindungsgemässen Verbindungen die Produktion von Zytokinen, Lymphokinen und auch Monokinen, deren Synthese durch entsprechend beeinflussbare Signaltransduktionselemente reguliert werden und daher für Krankheiten geeignet sind, wo Proteine als Vermittler am Krankheitsverlauf beteiligt sind, wie z.B. Asthma, Sepsis, Entzündungen, gewisse immunologische Prozesse und dergleichen.
Die Verbindungen der Formel I erzeugen eine nützliche Relaxation eines glatten Muskels der Bronchien, des Uterus, des Gefässsystems und dergleichen.
Diese Relaxation kann wie folgt nachgewiesen werden, indem in Segmenten, die dem Ileum eines 300 bis 400g schweren Meerschweinchens entnommen und in einem Organbad in Tyrode-Lösung bei 38°C und unter Begasung mit einem Gemisch aus 95% Sauerstoff und 5% Kohlendioxid bei einer Belastung von 1g inkubiert wurden mit synthetischem Leukotrien $D_4$ (in Form des Kaliumsalzes) oder Histamin, $PGE_{2a}$, Thromboxan Mimetica oder $BaCl_2$ (als depolarisierende Lösung)) Kontraktionen ausgelöst und diese isotonisch registriert werden. Das Ausmass der Hemmung der Kontraktionen durch die Prüfsubstanz wird nach einer Vorinkubation von 2 Minuten in Form der $IC_{50}$ ermittelt, wobei als $IC_{50}$ diejenige Konzentration bezeichnet wird, bei welcher die Testkontraktionen um 50% reduziert sind.
Die Verbindungen der Formel I sind ausserordentlich lang wirksam und in vivo ausgezeichnet wirksam. So kann z.B. im Bronchokonstriktions-Standardtest am Meerschweinchen bei Verabreichung einer Aerosollösung, enthaltend von etwa 0,00001 bis etwa 1Gewichtsprozent Prüfsubstanz, ein deutlicher $LTD_4$-antagonistischer Effekt beobachtet werden. In diesem Testmodell anästhetisiert man männliche, 400 bis 700g schwere, Meerschweinchen intraperitoneal mit 1,4g/kg Urethan und führt eine Polyethylenkanüle in die Vena jugularis ein. Eine zweite Polyethylenkanüle wird in die Trachea eingeführt. Mittels einer in die Speiseröhre eingeführten Kanüle, welche mit einem Statham-Druck-Transduktor verbunden ist, wird der Druck in der Speiseröhre aufgezeichnet. Das Tier wird in eine luftdicht verschliessbare Plexiglaskammer gelegt, die mit einer Fleisch'schen Röhre Nr.000 und einem Validyne-Transducer MP45-1 verbunden ist. Mit dieser Anordnung wird der Flow gemessen. Nach der chirurgischen Präparierung der Versuchstiere wartet man eine gewisse Zeit, damit sich die pulmonalen Funktionen stabilisieren können. Die Prüfsubstanz wird anschliessend gemäss dem nachfolgenden Protokoll verabreicht. Die Versuchstiere werden während einer Minute einer 1-prozentigen (Gewicht/Volumen) Aerosollösung der Prüfsubstanz oder destilliertem Wasser (zu Kontrollzwecken) ausgesetzt. Für alle Testverbindungen, welche durch Inhalation verabreicht werden, verwendet man ein Monaghan-UltraschallSprühgerät (Modell670), dessen Partikelgrösse sich zwischen 1 und 8Mikrometer, mit einem Hauptanteil von 3Mikrometer, bewegt. Wässrige Lösungen werden jeweils frisch hergestellt und mit einem On-stream drug vial in die Kammer des Sprühgeräts eingeführt. Der produzierte Sprühnebel wird den Versuchstieren über eine Glaskammer von 65ml Inhalt, die mit einer Kanüle mit der Trachea verbunden wird, verabreicht. Nach Ablauf der Behandlungszeit wird mit einem zweiten Monaghan-Ultraschall-Sprühgerät (Modell670) und über eine gleiche Glaskammer$LTD_4$ (0,3µg/ml) oder Histamin (0,01% w/v) während 2Minuten verabreicht. Es wird die Abnahme der Compliance in der 3.Minute nach $LTD_4$ oder Histamin-Applikation abgelesen, und zwar wird der Mittelwert von drei Tieren mit dem Mittelwert von drei Kontrolltieren verglichen und die prozentuale Hemmung der Compliance (%Hemmung) nach folgender Formel errechnet:

$$\% \text{ Hemmung} = 100 - \frac{(100 - \text{Compliance Präparat}) \cdot 100}{(100 - \text{Compliance Kontrolle})}$$

Werden unterschiedliche Wirkstoffkonzentrationen untersucht, so wird die prozentuale Hemmung für jede Konzentration aufgezeichnet, und zwar wird "log Konzentration" auf der Abszisse gegen "prozentuale Hemmung" auf der Ordinate aufgetragen. Die $IC_{50}$ wird dann durch lineare Regressionsanalyse ermittelt.

Die Verbindungen I und ihre pharmazeutisch verwendbaren Salze weisen weiterhin den spezifischen und therapeutisch sehr bedeutsamen Vorteil einer verhältnismässig langen Wirkungsdauer auf.

Bevorzugt sind im Rahmen der Erfindung Verbindungen der Formel I, worin $R_1$ und $R_2$ gleichzeitig Wasserstoff oder beide den gleichen Niederalkylrest bedeuten oder gemeinsam für 4- bis 5-gliedriges Niederalkylen stehen und ihre Salze in razemischer und chiraler Form.

Besonders bevorzugt sind im Rahmen der Erfindung Verbindungen der Formel I, worin $R_1$ und $R_2$ gleichzeitig Wasserstoff oder beide gleichzeitig einen Methyl- oder Ethylrest bedeuten, und ihre pharmazeutisch verwendbaren Salze in razemischer und chiraler Form.

Ganz besonders bevorzugt im Rahmen der Erfindung Verbindung der Formel I, worin $R_1$ und $R_2$ gleichzeitig Wasserstoff bedeuten und ihre pharmazeutisch verwendbaren Salze in razemischer und chiraler Form.

Namentlich bevorzugt im Rahmen der Erfindung ist das im Beispiel 1 genannte Razemat aus (5R)-5-(3-Formylamino-4-hydroxyphenyl)-3-[(2R)-1-(4-methoxyphenyl)prop-2-yl]-oxazolidin und (5S)-5-(3-Formylamino-4-hydroxyphenyl)-3-[(2S)-1-(4-methoxy phenyl)prop-2-yl]-oxazolidin oder die Enantiomeren in reiner Form oder ein pharmazeutisch verwendbaren Salz davon. Ebenfalls Gegenstand der vorliegenden Erfindung ist ein Verfahren zur Herstellung von Verbindungen der Formel I und ihrer Salze, z.B. dadurch gekennzeichnet, dass man eine Verbindung der Formel II

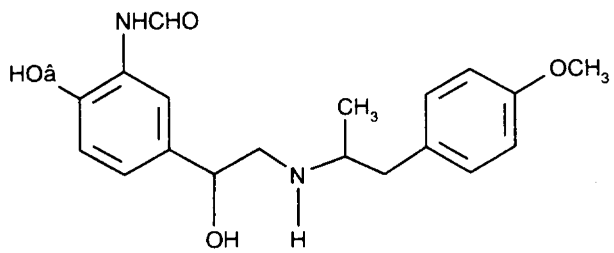

(II)

mit einer Verbindung der Formel III

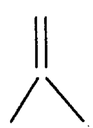

(III)

in freier und acetalisierter bzw. ketalisierter Form, worin $R_1$ und $R_2$ die unter der Formel I angegebenen Bedeutungen hat, umsetzt.

Die Kondensation der Verbindungen der Formel II mit Verbindungen der Formel III erfolgt in üblicher Weise in einem protischen oder aprotischen Lösungsmittel, wie z.B. eines aliphatischen Halogenkohlenwasserstoffes, wie in Dichloralkane, insbesondere Methylenchlorid, oder eines aliphatischen oder cycloaliphatischen Ethers, z.B. in Tetrahydrofuran oder auch Dioxan. Ferner kommen als Lösungsmittel beispielsweise Acetonitril, Ethanol und Toluol in Frage.

Die Substanzen werden bei einer Temperatur zwischen -10° bis +60°C, vorzugsweise zwischen 0°C bis +30°C, vorteilhafterweise in Gegenwart eines Katalysators, eines sauren Kondensationsmittels, beispielsweise eines Ammoniumsalzes, wie z.B. Ammoniumacetat miteinander umgesetzt. Das Ausgangsprodukt der Formel II ist bekannt und dessen Herstellung in der DE-PatentschriftNr.2,305,092 beschrieben.

In analoger Weise sind auch die Verbindungen der Formel III bekannt und als Allgemeingut in jedem Lehrbuch beschrieben.

Verfahrensgemäss erhältliche Verbindungen können in üblicher Weise in andere Verbindungen der Formel I überführt

werden.
Erhaltene Salze können in an sich bekannter Weise in die freien Verbindungen umgewandelt werden, z.B. durch Behandeln mit einer Base, wie einem Alkalimetallhydroxid, einem Metallcarbonat oder -hydrogencarbonat, oder einer anderen eingangs genannten salzbildenden Base bzw. mit einer Säure, wie einer Mineralsäure, z.B. mit Chlorwasserstoff, oder einer anderen eingangs genannten salzbildenden Säure.
Erhaltene Salze können in an sich bekannter Weise in andere Salze überführt werden, Säureadditionssalze z.B. durch Behandeln mit einem geeigneten Metallsalz, wie einem Natrium-, Barium- oder Silbersalz, einer anderen Säure in einem geeigneten Lösungsmittel, in welchem ein sich bildendes anorganisches Salz unlöslich ist und damit aus dem Reaktionsgleichgewicht ausscheidet, und Basensalze durch Freisetzung der freien Säure und erneute Versalzung.
Die Verbindungen der Formel I einschliesslich ihrer Salze, können auch in Form von Hydraten erhalten werden oder das zur Kristallisation verwendete Lösungsmittel einschliessen.
Infolge der engen Beziehung zwischen den neuen Verbindungen in freier Form und in Form ihrer Salze sind vorstehend und nachfolgend unter den freien Verbindungen und ihren Salzen sinn- und zweckgemäss gegebenenfalls auch die entsprechenden Salze bzw. freien Verbindungen zu verstehen.
Die Verbindungen I und ihre Salze können, je nach Wahl der Ausgangsstoffe und Arbeitsweisen, in Form eines der Diastereomerengemische, Razemate und der Enantiomeren oder als Gemische derselben vorliegen.
Die Trennung erhaltener Razemate in die einzelnen Enantiomere erfolgt durch Säulenchromatographie über eine chirale stationäre Phase.

Erhaltene Razemate lassen sich ferner nach bekannten Methoden in die optischen Antipoden zerlegen, beispielsweise durch Umkristallisation aus einem optisch aktiven Lösungsmittel, mit Hilfe von Mikroorganismen oder durch Umsetzung des erhaltenen Diastereomerengemisches bzw. Razemates mit einer optisch aktiven Hilfsverbindung, z.B. entsprechend der in Verbindungen der Formel I erhaltenen sauren, basischen oder funktionell abwandelbaren Gruppen mit einer optisch aktiven Säure, Base oder einem optisch aktiven Alkohol, in Gemische diastereomerer Salze bzw. funktioneller Derivate, wie Ester, Trennung derselben in die Diastereomeren, aus denen das jeweils gewünschte Enantiomere in der jeweils üblichen Weise freigesetzt werden kann. Dafür geeignete Basen, Säuren bzw. Alkohole sind beispielsweise optisch aktive Alkaloidbasen, wie Strychnin, Cinchonin oder Brucin, oder D- oder L-(1-Phenyl)ethylamin, 3-Pipecolin, Ephedrin, Amphetamin oder ähnliche synthetisch zugängliche Basen, optisch aktive Carbon- oder Sulfonsäuren, wie Chinasäure oder D- oder L-Weinsäure, D- oder L-Di-o-toluylweinsäure, D- oder L-Äpfelsäure, D- oder L-Mandelsäure, oder D- oder L-Camphersulfonsäure, bzw. optisch aktive Alkohole, wie Borneol oder D- oder L-(1-Phenyl)ethanol.
Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Schritte durchführt oder einen Ausgangsstoff in Form eines Salzes verwendet oder insbesondere unter den Reaktionsbedingungen bildet.

Die neuen Ausgangsstoffe, die speziell für die Herstellung der erfindungsgemässen Verbindungen entwickelt wurden, insbesondere die zu den eingangs als bevorzugt gekennzeichneten Verbindungen der Formel I führende Ausgangsstoffwahl, die Verfahren zu ihrer Herstellung und ihre Verwendung als Zwischenprodukte bilden ebenfalls einen Gegenstand der Erfindung.
Bei den erfindungsgemässen pharmazeutischen Präparaten, welche die erfindungsgemässe Verbindung oder pharmazeutisch verwendbaren Salze davon enthalten, handelt es sich um solche zur enteralen, wie oralen, ferner rektalen, und parenteralen Verabreichung an Warmblüter(n), wobei der pharmakologische Wirkstoff allein oder zusammen mit einem pharmazeutisch anwendbaren Trägermaterial enthalten ist. Die tägliche Dosierung des Wirkstoffes hängt von dem Alter des Patienten, Geschlecht und dem individuellen Zustand sowie von der Applikationsweise ab.
Die erfindungsgemässen Verbindungen können für die Verabreichung auf irgendeine geeignete Weise formuliert sein.
Gegenstand der Erfindung sind Arzneimittel, die mindestens eine Verbindung der Formel(I) oder eines ihrer physiologisch annehmbaren Salze enthalten und die für die Verwendung in der Human- oder Veterinärmedizin zubereitet sind.
Solche Mittel können zusammen mit physiologisch annehmbaren Trägern oder Exzipienten, gegebenenfalls mit zusätzlichen medizinischen Mitteln bzw. Arzneimitteln, vorliegen.
Die Verbindungen können in einer Form zubereitet sein, die für die Verabreichung durch Inhalation oder Einblasen oder für die orale, bukkale, parenterale, topische (einschliesslich nasale) oder rektale Verabreichung geeignet sind. Die Verabreichung durch Inhalation oder Einblasen ist bevorzugt.

Für die Verabreichung durch Inhalation werden die erfindungsgemässen Verbindungen zweckdienlich in Form einer Aerosol-Sprayrepräsentation von unter Druck stehenden Packungen unter Verwendung eines geeigneten Teibgases eingesetzt.
Solche Treibgase oder Treibgasgemische sind an sich für die Herstellung von pharmazeutischen Aerosolen bekannt, z.B. unsubstituierte gesättige Kohlenwasserstoffe, z.B. n-Propan, n-Butan oder Isobutan oder Gemische davon, oder partiell fluorierte oder vollständig fluorierte (perfluorierte) Kohlenwasserstoffe.

Partiell fluorierte Kohlenwasserstoffe leiten sich von aliphatischen Kohlenwasserstoffen mit vorzugsweise 1-4C-Atomen, z.B. Methan, Ethan, Propan, n-Butan oder Isobutan, oder cycloaliphatischen Kohlenwasserstoffen mit vorzugsweise 3 und 4C-Atomen, ab, z.B. Cyclopropan oder Cyclobutan, indem die Wasserstoffatome durch mindestens ein Fluoratom und vorzugsweise mindestens zwei Fluoratome so substituiert sind, dass mindestens ein Wasserstoffatom und damit eine Kohlenwasserstoffbindung im Molekül bestehen bleibt. Vollständig fluorierte (perfluorierte) Kohlenwasserstoffe leiten sich von den genannten aliphatischen Kohlenwasserstoffen mit 1-4C-Atomen und den genannten cycloaliphatischen Kohlenwasserstoffen mit 3 und 4C-Atomen durch Substitution der Wasserstoffatome durch entsprechende Fluoratome ab.

Geeignete partiell oder vollständig fluorierte Kohlenwasserstoffe sind z.B. Methanderivate mit 1-4, Ethanderivate mit 1-6, Propanderivate mit 1-8, n-Butanderivate mit 1-10, Cyclopropanderivate mit 1-6 und Cyclobutanderivate mit 1-8Fluoratomen. In diesen partiell oder vollständig fluorierten Kohlenwasserstoffen können die Wasserstoffe sich an verschiedenen Stellen des Kohlenwasserstoffgerüstes befinden. Für partiell fluorierte Kohlenwasserstoffe sind folgende Isomeriefälle möglich:
Ist nur ein Wasserstoffatom vorhanden, kann dieses in den Propan- und Butanderivaten endständig sein oder sich an einem Zwischenglied der C-Kette befinden.

Bei mehr als einem Wasserstoffatom sind für die Ethan-, Propan-, n-Butan-, Cyclopropan- und Cyclobutanderivate sowie für Kohlenwasserstoffe mit einer noch höheren Anzahl C-Atomen noch weitere Isomeriefälle möglich. Die Wasserstoffatome können teilweise oder vollständig endständig sein und sich teilweise oder ganz an einem oder an verschiedenen Zwischengliedern der C-Ketten befinden. "Gemischte" Isomeriefälle sind auch möglich, indem die Wasserstoffatome sich in unterschiedlicher Verteilung bei aliphatischen Derivaten am endständigen C-Atom und an denselben oder an verschiedenen Kohlenstoff-Zwischengliedern oder bei cycloaliphatischen Derivaten sich an denselben oder an verschiedenen Kohlenstoffringgliedern befinden.
Zur Abkürzung der üblichen Nomenklatur und zur Unterscheidung der genannten partiell fluorierten Kohlenwasserstoffe sowie auch der im folgenden genannten vollständig fluorierten Kohlenwasserstoffe sind Codebezeichnungen geläufig, die in Pharmazeutische Technologie, H.Sucker, P.Fuch, P.Speiser (Herausgeber), Thieme Verlag, D-7000Stuttgart1978, auf der Seite735 erläutert und ebenfalls auf FCKW anwendbar sind. Für die genannten zahlreichen Isomeriefälle sind Zusatzbezeichnungen mit den Buchstabena, b... üblich.
Als partiell fluorierte Kohlenwasserstoffe sind Tetrafluorethan (134 und 134a), Trifluorethan (143a), Difluorethan (152 und 152a) und Heptafluorpropan (227) bevorzugt.
Alternativ können die erfindungsgemässen Verbindungen für die Verabreichung durch Inhalation oder Einblasen in Form eines trockenen Pulvers, z.B. als Pulvergemisch aus der Verbindung und einem geeigneten Pulvergrundstoff, wie Lactose oder Stärke, vorliegen. Das Pulvergemisch kann in Dosiseinheitsform, z.B. in Form von Kapseln oder Patronen aus beispielsweise Gelatine, oder in Form von Blasenpackungen vorliegen, aus denen das Pulver mit Hilfe einer Inhalationsvorrichtung oder eines Einblasgeräts abgegeben werden kann.

Für die orale Verabreichung können die pharmazeutischen Mittel in Form von beispielsweise Tabletten, Kapseln, Pulvern, Lösungen, Sirupen oder Suspensionen vorliegen, die nach bekannten Verfahren mit annehmbaren Verdünnungsmitteln bzw. Arzneimittelträgerstoffen hergestellt werden. Für die bukkale Verabreichung kann das Mittel in Form von Tabletten, Tropfen oder Lutschbonbons vorliegen, die auf bekannte Weise zubereitet werden.

Die erfindungsgemässen Verbindungen können auch für die parenterale Verabreichung verwendet werden. Zubereitungen für die Injektion können in Dosiseinheitsform in Ampullen oder in Mehr-Dosis-Behältern mit zugegebenen Konservierungsmitteln vorliegen. Die Zubereitungen können in Form von Suspensionen, Lösungen oder Emulsionen in öligen oder wässrigen Vehikeln vorliegen und können Zubereitungshilfsmittel, wie Suspensions-, Stabilisations-, und/oder Dispersionsmittel, enthalten. Alternativ kann der aktive Bestandteil in Pulverform für die Rekonstitution mit einem geeigneten Träger, z.B. sterilem, pyrogenfreiem Wasser, vor der Verwendung vorliegen.

Für die topische Verabreichung können die erfindungsgemässen, pharmazeutischen Mittel in Form von Salben, Lotionen oder Cremen, die in an sich bekannter Weise zubereitet werden, z.B. mit einem wässrigen oder öligen Grundstoff, im allgemeinen durch Zugabe geeigneter Verdickungsmittel und/oder Lösungsmittel, vorliegen. Für die nasale Anwendung können die Mittel in Form eines Sprays vorliegen, welcher beispielsweise als wässrige Lösung oder Suspension oder als Aerosol unter Verwendung geeigneter Treibmittel zubereitet wurde.
Die erfindungsgemässen Verbindungen können ebenfalls in rektalen Mitteln, wie Suppositorien oder Retentionsklistieren, vorliegen, z.B. solchen, die bekannte Suppositoriengrundstoffe, wie Kakaobutter oder andere Glyceride, enthalten.
Wenn die pharmazeutischen Mittel für die orale, bukkale, rektale oder topische Verabreichung verschrieben werden, können sie in an sich bekannter Weise mit Formen, die eine kontrollierte bzw. verzögerte Freigabe ermöglichen, assoziiert sein.
Eine vorgeschlagene, tägliche Dosismenge an aktiver Verbindung für die Behandlung von Menschen beträgt bei oraler Applikation 10 bis 500μg, wobei diese geeigneterweise in Form einer einzigen Dosis verabreicht werden kann. Die genaue, verwendete Dosis wird natürlich von dem Alter und dem Zustand des Patienten und dem Verabreichungsweg abhängen. Geeignete Dosierungen betragen für die Verabreichung durch Inhalation (Aerosole) oder nasale Applikation, 1-200μg, für die rektale Verabreichung 10 bis 500μg, für die intravenöse Verabreichung 0,01 bis 100μg und für die topische Verabreichung 1 bis 1000μg.

Die nachfolgenden Beispiele dienen zur Illustration der Erfindung; Temperaturen sind in Celsiusgraden, Drucke in mbar angegeben.

Beispiel 1:

Zu einer Suspension von 2,75g 2'-Hydroxy-5'-[(RS)-1-hydroxy-2-[[(RS)-p-methoxy-α-methyl-phenylethyl]-amino]-ethyl]-formanilid (razemisches Gemisch aus R,R- und S,S-Enantiomeren) in 80ml Methylenchlorid setzt man unter Rühren bei Raumtemperatur 1,0 ml 36,5%-igen wässrigen Formaldehyd zu. Die Mischung wird 30Minuten gerührt, wobei Lösung eintritt. Die Lösung wird weitere 15Stunden bei 26°gerührt. Die ausgeschiedenen Kristallewerden abfiltriert, mit wenig Methylenchlorid gewaschen und unter 0,1mbar bei 40° während 15Stunden getrocknet. Nach Umkristallisation aus Ethylacetat schmilzt das Razemat von (5R)-5-(3-Formylamino-4-hydroxyphenyl)-3-[(2R)-1-(4-methoxyphenyl)prop-2-yl]-oxazolidin und (5S)-5-(3-Formylamino-4-hydroxyphenyl)-3-[(2S)-1-(4-methoxyphenyl)prop-2-yl]-oxazol idin bei 87-92°, [$^1$H-NMR (400 MHZ, $CDCl_3$: 8,27 (d, 1H), 7,75 (b, 1H), 7,31 (d, 1H), 7,10 (m, 2H), 7,08 (d, d, 1H), 6,93 (d, 1H), 6,85 (m, 2H), 5,00 (t, 1H), 4,63 (AB-System, 2H), 3,81 (S, 3H), 3,43 und 2,80 (d, d/d, d, 2H), 2,81 (m, 1H), 2,93 und 2,51 (d, d/d, d, 2H). 1,04 (d, 3H)].

Beispiel 2:

Eine Mischung von 2,0g 2'-Hydroxy-5'-[(R,S)-1-hydroxy-2-[[(R,S)-p-methoxy -α-methyl-phenylethyl]-amino]-ethyl]-formanilid (razemisches Gemisch aus R,R- und S,S-Enantiomeren), 200ml 2,2-Dimethoxypropan und 6,0g Ammoniumacetat in 140ml Aceton wird unter Rühren während 15Stunden unter Rückfluss erhitzt, abgekühlt und unter 11mbar zur Trockene eingeengt. Den Rückstand versetzt man mit 10,0g Kaliumhydrogencarbonat, 50ml Eis-Wasser und 50ml Methylenchlorid. Nach kräftigem Schütteln wird die organische Phase abgetrennt, mit 10ml 2-n Kaliumhydrogencarbonat und mit 10ml Sole gewaschen, über Magnesiumsulfat getrocknet und unter 11 mbar bei 40° eingedampft. Der Rückstand wird an 70g Silikagel flash-chromatographiert. Die Fraktionen 1-8, eluiert mit je 100ml Methylenchlorid-Methanol (98:2), werden verworfen. Die Fraktionen 9-14, eluiert mit je 100ml Methylenchlorid-Methanol (98:2), werden vereinigt und unter 11mbar bei 40° eingedampft. Der Rückstand, das razemische Gemisch aus 2,2-Dimethyl-(5R)-5-(3-formylamino-4-hydroxyphenyl)-3-[(2R)-1-(4-methoxyphenyl)prop-2-yl]-oxazolidin und 2,2-Dimethyl-(5S)-5-(3-formylamino-4-hydroxyphenyl)-3-[(2S)-1-(4-methoxyphenyl)prop-2-yl]-oxazolidin liegt als amorphes Pulver vor. [$^1$NMR(400 MHZ, CD $Cl_3$; 8,42 (b, 1H), 8,29 (b, 1H), 7,66 (d, b, 1H), 7,10 (m, 2H), 6,87 (m, 2H), 6,81 (d, d, 1H), 6,57 (d, 1H), 4,84 (t, 1H), 3,82 (s, 3H), 3,10 ... 2,66 (m, 5H), 1,28 und 1,11 (2s, 2x3H), 1,18 (d, 3H)].

Beispiel 3:

Die RR- und SS-Enantiomeren des nach Beispiel 1 erhaltenen razemischen Gemisches werden wie folgt erhalten: 20μl einer 0,1% Lösung vom razemischen Gemisch nach Beispiel 1 erhalten, werden auf eine chirale "CHIRALCEL OJ" (Daicel Chemical Industries, Japan) HPLC Säule eingespritzt. Das Trägermaterial besteht aus einem mit para-Methylbenzoylcellulose belegten Kieselgel. Bei einem Durchfluss von 1 ml/min und mit einem aus Hexan (85 Vol%) und 2-Propanol (15 Vol%) bestehenden Fliessmittel werden die Enantiomeren mit einem Trennfaktor α=1.27 getrennt.

Beispiel 4:

20μl einer 1% Lösung vom razemischen Gemisch nach Beispiel1 erhalten werden auf eine chirale meta-Methylbenzoylcellulose (Herstellung gemäss Patent EP-A-0316270) HPLC Säule eingespritzt. Bei einem Durchfluss von 1 ml/min und mit einem aus Hexan (85Vol%) und 2-Propanol (15Vol%) bestehenden Fliessmittel werden die Enantiomeren mit einem Trennfaktor α=1.18 getrennt.

Beispiel 5:

Zu einer Lösung von 99.5 mg (-)-2'-Hydroxy-5'-(R)-1-hydroxy-2-[[(R)-p-methoxy-α-methyl-phenethyl]amino]-ethyl]-formanilid (R,R-Enantiomeres) in 3 ml Methylenchlorid setzt man unter Rühren bei Raumtemperatur 22 μl 37%-igen wässrigen Formaldehyd zu. Die Mischung wird 15 Stunden bei 5-15° gerührt, wobei Lösung eintritt.Man verdünnt mit 3 ml Methylenchlorid, wäscht die Methylenchloridphase mit Wasser (2 x 2 ml),trocknet sie über Magnesiumsulfat und dampft diese bei 20 mbar und 40° ein. Der Rückstand wird unter 0.1 mbar bei 25° während 15 Stunden getrocknet. Auf diese Weise erhält man das (-)-(5R)-5-(3-Formylamino-4-hydroxyphenyl)-3-[(2R)-1 -(4-methoxyphenyl)prop-2-yl]-oxazolidin als weissen amorphen Feststoff. [$^1$H-NMR (400 MHZ, $CD_2Cl_2$: 8.24 (d,1H), 7.70 (b,1H), 7.21 (d,1H), 7.10 (m,2H), 7.09 (d,d,1 H), 6.93 (d.1H), 6.83 (m,2H), 4.94 (+,1H), 4,57 (AB,2H), 3.78 (s,3H), 3.40 und 2.75 (2d,d,2H), 2.88 und 2,50 (2d,d,2H), 2.79 (m,1H),1.00 (d,3H)].

$[\alpha]^{20}_D$ = -13.1 ± 1.9° (c = 0.518, MeOH)

Beispiel 6:

Zu einer Lösung von 151 mg (+)-2'-Hydroxy-5'-(S)-1-hydroxy-2-[[(S)-p-methoxy-α-methyl-phenethyl]-amino]-ethyl]-formanilid (S,S-Enantiomeres) in 4.5 ml Methylenchlorid setzt man bei 5-15° unter Rühren 33.38 μl 37%-igen Formaldehyd zu. Die Mischung wird 15 Stunden bei 5-15° gerührt, wobei Lösung eintritt. Man verdünnt mit 4 ml Methylenchlorid, wäscht die Methylenchloridphase mit Wasser (2 x 3 ml), trocknet sie über Magnesiumsulfat und dampft diese bei 15 mbar und 40° ein. Der Rückstand wird unter 0.1 mbar bei 25° während 15 Stunden getrocknet. Man erhält das (+)-(5S)-5-(3-Formylamino-4-hydroxyphenyl)-3-[(2S)-1-(4-methoxyphenyl)prop-2-yl]-oxazolidin als amorphes Pulver. [$^1$H-NMR (400 MHZ, $CD_2Cl_2$)]: Identisch mit dem Spektrum der Verbindung aus Beispiel 5.
$[\alpha]^{20}_D$ = + 17.3 ± 1.8° (c = 0.550, MeOH)

**Analytische Bestimmung der optischen Reinheit der Enantiomeren**

Die analytische Bestimmung der optischen Reinheit der Enantiomeren (R,R) (Beispiel 5) und (S,S) (Beispiel 6) erfolgte durch HPLC auf der chiralen Säule (0.46 cm x 25 cm) Chiracel OJ (Daicel Chemical Industries, Japan) mit einem aus Hexan (85 Vol.%) und Ethanol (15 Vo.%) bestehenden Gemisch als Fliessmittel und bei einem Durchfluss von 1 mil/min. Die Chromatographie-Anlage besteht aus einer Pumpe (Gilson,Modell 303), einem Autosampler (Shimadzu SLC-6B9), einem variablen UV Detektor (Perkin-Elmer LC-95) und einem Datenerfassungssystem (Metrohm AG, IC-Metrodata 714). 20 Microliter einer 0,1 %-igen Lösung (in Ethanol) der einzelnen hergestellten Enantiomeren wurden eingespritzt und ergaben die folgenden Resultate (Detektion bei 240 nm): (+) (S,S) Enantiomer (Verbindung aus Beispiel 6), Retentionzeit 93,31 min, ee > 99,9%; (-) (R,R) Enantiomer (Verbindung aus Beispiel 5), Retentionszeit 115,89 min, ee > 99,9%.

**Patentansprüche**

1. Verbindungen der Formel(I)

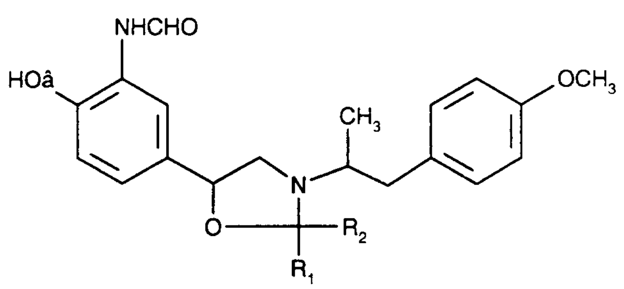

(I)

in welcher $R_1$ und $R_2$ gleichzeitig Wasserstoff, oder beide den gleichen Niederalkylrest mit 1-4 C-Atomen bedeuten oder gemeinsam für 4- bis 7-gliedriges Niederalkylen stehen, und ihre Salze in razemischer und chiraler Form.

2. Verbindungen der Formel I gemäss Anspruch 1, worin $R_1$ und $R_2$ gleichzeitig Wasserstoff oder beide den gleichen Niederalkylrest mit 1-4 C-Atomen bedeuten oder gemeinsam für 4- bis 5-gliedriges Niederalkylen stehen und ihre Salze in razemischer und chiraler Form.

3. Verbindungen der Formel I gemäss Anspruch 1, worin $R_1$ und $R_2$ gleichzeitig Wasserstoff oder beide gleichzeitig einen Methyl- oder Ethylrest bedeuten, und ihre pharmazeutisch verwendbaren Salze in razemischer und chiraler Form.

4. Verbindungen der Formel I gemäss Anspruch 1, worin $R_1$ und $R_2$ gleichzeitig Wasserstoff bedeuten und ihre pharmazeutisch verwendbaren Salze in razemischer und chiraler Form.

5. Das Razemat aus (5R)-5-(3-Formylamino-4-hydroxyphenyl)-3-[(2R)-1-(4-methoxyphenyl)prop-2-yl]-oxazolidin und (5S)-5-(3-Formylamino-4-hydroxyphenyl)-3-[(2S)-1-(4-methoxyphenyl)prop-2-yl]-oxazolidin oder ein pharmazeutisch verwendbares Salz davon.

6. (5R)-5-(3-Formylamino-4-hydroxyphenyl)-3-[(2R)-1-(4-methoxyphenyl)prop-2-yl]-oxazolidin oder ein pharmazeutisch verwendbares Salz davon.

7. (5S)-5-(3-Formylamino-4-hydroxyphenyl)-3-[(2S)-1 -(4-methoxyphenyl)prop-2-yl]-oxazolidin oder ein pharmazeutisch verwendbares Salz davon.

8. Verbindungen gemäss einem der Ansprüche 1-7 zur Anwendung in einem Verfahren zur therapeutischen Behandlung des menschlichen oder tierischen Körpers.

9. Pharmazeutische Präparate, neben üblichen pharmazeutischen Hilfsstoffen als pharmazeutischen Wirkstoff enthaltend eine Verbindung gemäss einem der Ansprüche 1 bis 7 in freier Form oder in pharmazeutisch verwendbarer Salzform.

10. Verfahren zur Herstellung neuer Verbindungen der Formel I gemäss Anspruch 1, dadurch gekennzeichnet, dass man
eine Verbindung der Formel II

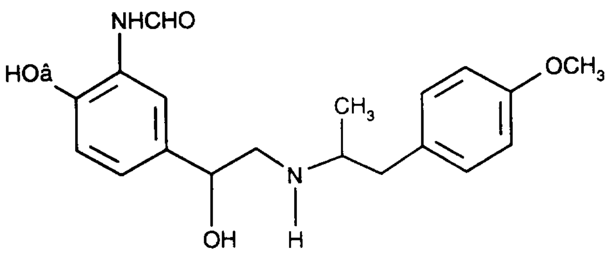

(II)

mit einer Verbindung der Formel III

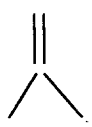

(III)

in freier und acetalisierter bzw. ketalisierter Form, worin $R_1$ und $R_2$ die unter der Formel I angegebenen Bedeutungen hat, umsetzt und gewünschtenfalls ein erfindungsgemäss erhältliches Isomerengemisch in die individuellen Isomeren auftrennt und das gewünschte Isomere isoliert und/oder eine erfindungsgemäss erhältliche freie Verbindung in ein Salz oder ein erfindungsgemäss erhältliches Salz in die freie Verbindung oder in ein anderes Salz überführt.

11. Verfahren nach Anspruch 10, dadurch gekennzeichnet, dass man ein erhaltenes Razemat einer Verbindung der Formel I in die einzelnen Enantiomeren auftrennt.

12. Verfahren nach Anspruch 11, dadurch gekennzeichnet, dass man ein erhaltenes Razemat einer Verbindung der Formel I über eine chirale stationäre Phase säulenchromatographisch auftrennt.

## Claims

1. A compound of formula (I)

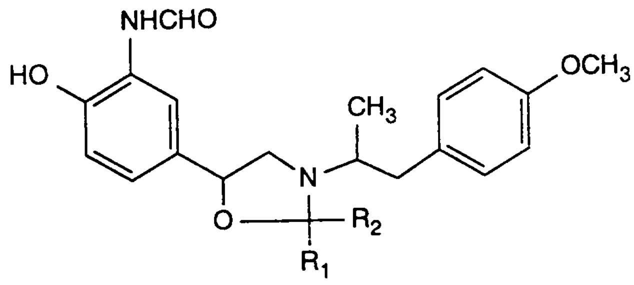

(I)

wherein $R_1$ and $R_2$ are simultaneously hydrogen or are both the same lower alkyl radical having from 1 to 4 carbon atoms, or together form 4- to 7-membered lower alkylene, or a salt thereof in racemic or chiral form.

2. A compound of formula I according to claim 1, wherein $R_1$ and $R_2$ are simultaneously hydrogen or are both the same lower alkyl radical having from 1 to 4 carbon atoms, or together form 4- or 5-membered lower alkylene, or a salt thereof in racemic or chiral form.

3. A compound of formula I according to claim 1, wherein $R_1$ and $R_2$ are simultaneously hydrogen or are both simultaneously a methyl or ethyl radical, or a pharmaceutically acceptable salt thereof in racemic or chiral form.

4. A compound of formula I according to claim 1, wherein $R_1$ and $R_2$ are simultaneously hydrogen, or a pharmaceutically acceptable salt thereof in racemic or chiral form.

5. The racemate of (5R)-5-(3-formylamino-4-hydroxyphenyl)-3-[(2R)-1-(4-methoxyphenyl)prop-2-yl]-oxazolidine and (5S)-5-(3-formylamino-4-hydroxyphenyl)-3-[(2S)-1-(4-methoxyphenyl)prop-2-yl]-oxazolidine or a pharmaceutically acceptable salt thereof.

6. (5R)-5-(3-Formylamino-4-hydroxyphenyl)-3-[(2R)-1-(4-methoxyphenyl)prop-2-yl]-oxazolidine or a pharmaceutically acceptable salt thereof.

7. (5S)-5-(3-Formylamino-4-hydroxyphenyl)-3-[(2S)-1-(4-methoxyphenyl)prop-2-yl]-oxazolidine or a pharmaceutically acceptable salt thereof.

8. A compound according to any one of claims 1 to 7 for use in a method for the therapeutic treatment of the human or animal body.

9. A pharmaceutical composition comprising, in addition to customary pharmaceutical excipients, a compound according to any one of claims 1 to 7 in free form or in pharmaceutically acceptable salt form as pharmaceutical active ingredient.

10. A process for the preparation of a novel compound of formula I according to claim 1, which comprises reacting a compound of formula II

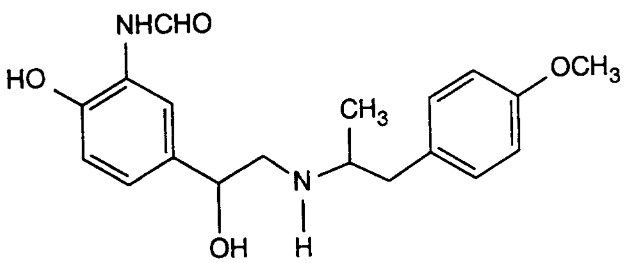

(II)

with a compound of formula III

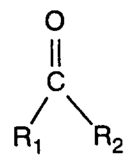

(III)

in free form or in acetalised or ketalised form wherein $R_1$ and $R_2$ are as defined for formula I, and, if desired, separating a mixture of isomers obtainable according to the invention into the individual isomers and isolating the desired isomer and/or converting a free compound obtainable according to the invention into a salt or converting a salt obtainable according to the invention into the free compound or into a different salt.

**11.** A process according to claim 10 wherein a resulting racemate of a compound of formula I is separated into the individual enantiomers.

**12.** A process according to claim 11 wherein a resulting racemate of a compound of formula I is separated by way of column chromatography over a chiral stationary phase.

## Revendications

**1.** Les composés de formule (I)

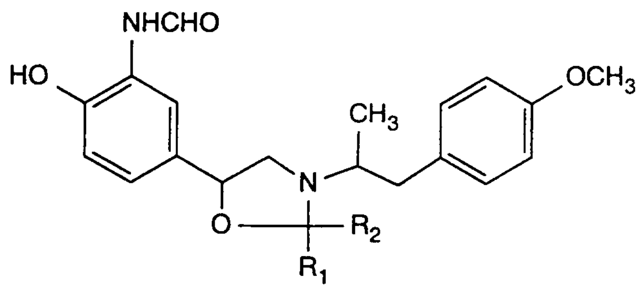

(I)

où $R_1$ et $R_2$ signifient simultanément l'hydrogène, ou bien signifient tous les deux le même reste alkyle inférieur ayant de 1 à 4 atomes de carbone ou bien forment ensemble un reste alkylène inférieur ayant de 4 à 7 chaînons, et leurs sels sous forme racémique et chirale.

**2.** Les composés de formule I selon la revendication 1, où $R_1$ et $R_2$ signifient simultanément l'hydrogène ou bien signifient tous les deux le même reste alkyle inférieur ayant de 1 à 4 atomes de carbone ou bien forment ensemble un reste alkylène inférieur ayant de 4 à 5 chaînons et leurs sels sous forme racémique et chirale.

**3.** Les composés de formule I selon la revendication 1, où $R_1$ et $R_2$ signifient simultanément l'hydrogène ou bien signifient tous les deux simultanément un reste méthyle ou éthyle, et leurs sels pharmaceutiquement acceptables, sous forme racémique et chirale.

**4.** Les composés de formule I selon la revendication 1, où $R_1$ et $R_2$ signifient simultanément l'hydrogène et leurs sels pharmaceutiquement acceptables sous forme racémique et chirale.

**5.** Les racémate de la (5R)-5-(3-formylamino-4-hydroxyphényl)-3-[(2R)-1-(4-méthoxyphényl)prop-2-yl]-oxazolidine et de la (5S)-5-(3-formylamino-4-hydroxyphényl)-3-[(2S)-1-(4-méthoxyphényl)prop-2-yl]-oxazolidine ou un de ses sels pharmaceutiquement acceptables.

**6.** La (5R)-5-(3-formylamino-4-hydroxyphényl)-3-[(2R)-1-(4-méthoxyphényl)prop-2-yl]-oxazolidine ou un de ses sels pharmaceutiquement acceptables.

**7.** La (5S)-5-(3-formylamino-4-hydroxyphényl)-3-[(2S)-1-(4-méthoxyphényl)prop-2-yl]-oxazolidine ou un de ses sels

pharmaceutiquement acceptables.

**8.** Les composés selon l'une des revendications 1 à 7, pour l'utilisation dans un procédé pour le traitement thérapeutique d'un corps humain ou animal.

**9.** Des compositions pharmaceutiques contenant, outre les excipients pharmaceutiques habituels, un composé selon l'une des revendications 1 à 7, sous forme libre ou sous forme d'un sel pharmaceutiquement acceptable, comme substance active pharmaceutique.

**10.** Un procédé de préparation des nouveaux composés de formule I selon la revendication 1, caractérisé en ce que on fait réagir un composé de formule II

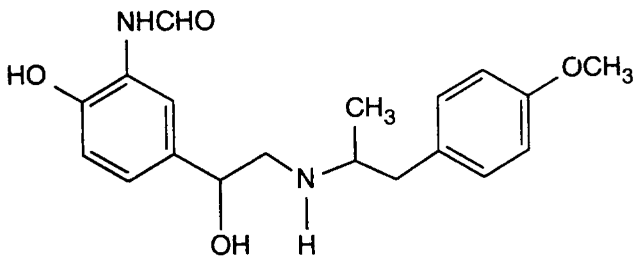

(II)

avec un composé de formule III

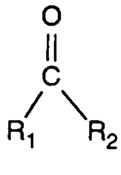

(III)

sous forme libre et sous forme acétalisée ou cétalisée, où $R_1$ et $R_2$ ont les significations indiquées sous la formule I et, éventuellement, on sépare un mélange d'isomères obtenu selon l'invention en isomères individuels et on isole l'isomère voulu et/ou on transforme un composé libre obtenu selon l'invention en un sel ou on transforme un sel obtenu selon l'invention en un composé libre ou en un autre sel.

**11.** Un procédé selon la revendication 10, caractérisé en ce qu'on sépare un racémate obtenu d'un composé de formule I en énantiomères individuels.

**12.** Un procédé selon la revendication 11, caractérisé en ce qu'on sépare un racémate obtenu d'un composé de formule I par chromatographie sur colonne avec phase stationnaire chirale.